# EUROPÄISCHE PATENTSCHRIFT

(11) **EP 1 065 201 B1**
(45) Veröffentlichungstag und Bekanntmachung des Hinweises auf die Patenterteilung: **14.08.2002**
(21) Anmeldenummer: 00111932.0
(22) Anmeldetag: 15.06.2000
(51) Int. Cl.: C07D 239/10, C07D 233/32, C07D 233/34

(54) **Verfahren zur Herstellung von im wesentlichen Ameisensäure-freien N-Alkyl-N'-methylalkylenharnstoffen**
Process for the preparation of, in the main, formic acid-free n-alkyl-n'-methylalkyleneurea
Procédé de préparation de n-alkyl-n'-méthylalkylèneurées en substance exemptes d'acide formique

(30) Priorität: 28.06.1999 DE 19929602
(43) Veröffentlichungstag der Anmeldung: 03.01.2001
(73) Patentinhaber: BASF AKTIENGESELLSCHAFT, 67056 Ludwigshafen (DE)
(72) Erfinder: Kramer, Andreas, Dr., 67251 Freinsheim (DE); Melder, Johann-Peter, Dr., 67459 Böhl-Iggelheim (DE); Rütter, Heinz, Dr., 67126 Hochdorf-Assenheim (DE); Riewe, Günter, 67125 Darmstadt-Schauernheim (DE); Siegel, Wolfgang, Dr., 67117 Limburgerhof (DE); Weyer, Hans-Jürgen, Dr., 67240 Bobenheim-Roxheim (DE)
(74) Vertreter: Isenbruck, Günter, Dr.

(56) Entgegenhaltungen:
- EP-A- 0 280 781
- EP-A- 0 356 973

## Beschreibung

Die Erfindung betrifft ein Verfahren zur Herstellung von N-Alkyl-N'-methylalkylenharnstoffen und insbesondere die Herstellung von N, N'-dimethylpropylen- und ethylenharnstoff, die im wesentlichen frei sind von Ameisensäure.

N, N'-dimethylpropylenharnstoff (DMPU) und der analoge N, N'-dimethyl-ethylenharnstoff (DMEU) haben Bedeutung als polare aprotische Speziallösungsmittel in Agro- und Pharmasynthesen. Besonders in Reaktionen mit Carbanionen oder Carbanionenäquivalenten können DMPU oder DMEU das cancerogene Hexamethylphosphorsäuretriamid ersetzen. Außerdem werden diese N, N'-dimethylalkylenharnstoffe in der Verfahrenstechnik z.B. bei extraktiven Destillationen zur Abtrennung von Cyclohexen aus einem Gemisch mit Benzol und Cyclohexan eingesetzt.

Für die Herstellung solcher tetrasubstituierter Harnstoffe hat insbesondere die sogenannte "Leuckart-Wallach Reaktion" Bedeutung erlangt, bei der Propylenharnstoff mit Aldehyden in Gegenwart von Ameisensäure umgesetzt wird, wie die EP-A-0 301 270 beschreibt.

In der EP-A-0 280 781 wird die Herstellung von N-alkyl-N'-methylalkylenhamstoffen und insbesondere von N, N'-dimethylalkylenharnstoffen, wie DMPU aus Propylenharnstoff und Formaldehyd in Gegenwart von Ameisensäure nach der sogenannten "Eschweiler-Clark"-Variante beschrieben.

Problematisch ist dabei jedoch die möglichst vollständige destillative Abtrennung der für diese Umsetzung erforderlichen Ameisensäure. Gemäß der EP-A-0 280 781 werden zur vollständigen Entfernung der Ameisensäure entweder Basen, wie Alkali- oder Erdalkalihydroxide oder -carbonate, zugesetzt oder ein niederer Alkohol sowie eine katalytische Menge einer Mineralsäure, die zur Veresterung der noch vorhandenen restlichen Ameisensäure dient. Der Ester wird dann destillativ entfernt.

Eine andere Möglichkeit zur im wesentlichen vollständigen Abtrennung der Ameisensäure nach der Umsetzung wird in der EP-A-0 356 973 beschrieben. Dort wird die nach der Umsetzung noch im Reaktionsgemisch enthaltene Ameisensäure mittels eines Katalysatorsystems aus einem tertiären Amin und einem Kupfersalz, wie CuCl, einer thermischen Zersetzungsreaktion bei Temperaturen von etwa 120 bis 250°C unterworfen.

Ausgehend von diesem Stand der Technik lag der Erfindung die Aufgabe zugrunde, ein Verfahren zu entwickeln, das die Herstellung von im wesentlichen Ameisensäure-freien N-alkyl-N'-methylalkylenharnstoffen und insbesondere die Herstellung von DMPU aus Propylenharnstoff und Formaldehyd unter "Eschweiler-Clark"- Bedingungen ohne aufwendige Aufarbeitungsschritte ermöglicht.

Diese Aufgabe wird gelöst durch ein Verfahren zur Herstellung von N-alkyl-N'methylharnstoffen, die die nachfolgend wiedergegebene Formel aufweisen 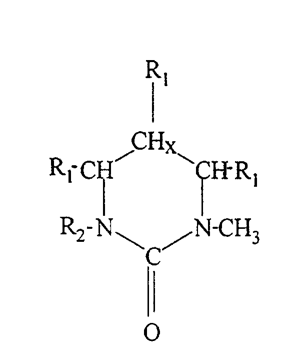
mit R₁ = H oder CH₃, R₂ = CₙH₂ₙ₊₁ mit n = 1-4 und x = 0 oder 1,

aus den entsprechenden Alkylenharnstoffen, durch Umsetzung mit monomerem oder polymerisiertem Formaldehyd in Gegenwart von Ameisensäure, bei dem ein im wesentlichen Ameisensäure-freies Endprodukt dadurch erhalten wird, daß das bei der Umsetzung erhaltene Gemisch aus N-Alkyl-N'-methylalkylenharnstoff und Ameisensäure dem oberen Bereich einer Destillationskolonne zugeführt wird, ohne weitere Zusätze destilliert und im unteren Bereich der Kolonne im wesentlichen Ameisensäure-freier N-Alkyl-N'-methylalkylenharnstoff ent-nommen wird, wobei der Druck im oberen Bereich der Kolonne höher eingestellt wird als im unteren Bereich der Kolonne und der Differenzdruck zwischen dem oberen und dem unteren Bereich der Kolonne 10 bis 100 mbar beträgt und die Temperatur im unteren Bereich der Kolonne höher als im oberen Bereich der Kolonne eingestellt wird, mit einer Temperaturdifferenz zwischen dem oberen und dem unteren Bereich der Kolonne von 40°C bis 210°C. Durch den erfindungsgemäßen Aufbau eines vorbestimmten Konzentrationsprofiles in der Kolonne kann die Ameisensäure im oberen Kolonnenteil gehalten werden, ohne daß es zum Durchbrechen der Säure in dem als Produkt dem unteren Kolonnenteil entnommenen N-Alkyl-N'-methylalkylenharnstoff kommt.

Vorzugsweise beträgt der Differenzdruck zwischen dem oberen Bereich der Kolonne, d.h. dem Kolonnenkopf, und dem unteren Bereich der Kolonne, d.h. dem Kolonnensumpf, 20 bis 40 mbar und die Temperaturdifferenz 80°C bis 150°C.

Der N-Alkyl-N'-methylalkylenharnstoff kann dem unteren Bereich der Kolonne gasförmig entnommen werden. Die verwendete Kolonne weist vorzugsweise eine Packung mit zumindest etwa 30 theoretischen Böden auf.

Dabei beträgt der Anteil an Ameisensäure, der nach der Umsetzung noch in dem Reaktionsgemisch enthalten ist, bis zu 35 Gew.-%. Unter "im wesentlichen Ameisensäure-frei" wird im Sinne dieser Erfindung ein Ameisensäuregehalt im Endprodukt von weniger als 0,1 % verstanden. Die eigentliche Umsetzung der entsprechenden Alkylenharnstoffe mit Formaldehyd kann unter Verwendung von monomerem oder polymerisiertem Formaldehyd, wie Paraformaldehyd, durchgeführt werden.

Gemäß einer bevorzugten Ausführungsform des erfindungsgemäßen Verfahrens wird der entsprechende Alkylenharnstoff mit dem Formaldehyd und der Ameisensäure bei einer Temperatur von 70 bis 150°C reagieren gelassen. Dabei wird die Rückflußtemperatur des Reaktionsgemisches bei Normaldruck, die einer Temperatur von etwa 100 bis 110°C entspricht, besonders bevorzugt. Anschließend kann zunächst das bei dieser Umsetzung entstehende Gemisch aus Ameisensäure und Wasser abdestilliert werden. Dann wird in einem weiteren Schritt das gewünschte Produkt von dem noch verbliebenen erheblichen Anteil an Ameisensäure befreit.

Besonders bewährt hat sich eine zweistufige Verfahrensführung, bei welcher in einem ersten Schritt bereits der erfindungsgemäß im wesentlichen Ameisensäure-freie N-Alkyl-N'-methylalkylenharnstoff erhalten und in einem zweiten Schritt eine Reindestillation zur Abtrennung von Neben- und Hochsiedern durchgeführt wird. Dadurch wird die Raum-Zeit-Ausbeute erhöht.

Besonders bevorzugt wird das Verfahren zur Herstellung von N, N'-dimethylpropylen- und ethylenharnstoff verwendet.

### Erstes Beispiel

### a) Einstufige Herstellung von DMPU

Ein 4 m³ Rührkessel wird mit 2000 kg Ameisensäure, 875 kg Propylenharnstoff und 650 kg Paraformaldehyd befüllt und langsam unter Rückflußkühlung zum Sieden erhitzt. Nach etwa 8 Stunden bei ca. 100°C wird unter Normaldruck bis zu einer Kesseltemperatur von 130°C ein Gemisch aus Ameisensäure und Wasser abdestilliert, das wieder in die Synthese zurückgeführt werden kann. Der Reaktorinhalt, der jeweils aus etwa 63 % DMPU, 30 % Ameisensäure, 1,5 % Wasser, 2 % Leichtsieder und 3,5 % Hochsieder besteht, wird abgekühlt und der Destillation zugeführt. Dieses nach der Umsetzung erhaltene Gemisch wird im folgenden als DMPU-Rohaustrag bezeichnet.

### b)

Der bei der Umsetzung, wie unter a) beschrieben, erhaltene DMPU-Rohaustrag wird kontinuierlich in eine Destillationskolonne mit Gewebepackung, die etwa 60 theoretische Böden aufweist, im oberen Bereich der Kolonne, etwa bei dem 45. Boden eingespeist und der im wesentlichen Ameisensäure-freie N, N'-dimethylpropylenharnstoff gasförmig über einen Seitenabzug im unteren Teil der Kolonne im Bereich des 10. Bodens entnommen.

Die Reaktionsparameter wurden so eingestellt, daß der Druck am Kolonnenkopf etwa 200 mbar und der Differenzdruck über die Kolonne etwa 30 mbar betrug. Die Temperatur betrug im Bereich des Zulaufes etwa 170°C, am Kolonnenkopf 107 bis 110°C, im Bereich des 10. Bodens etwa 190°C und im Sumpf 200 bis 202°C.

Das über den Seitenabzug entnommene Endprodukt wies folgende Zusammensetzung auf:
> 98 % DMPU, <0,1 % Ameisensäure, <0,1 % Wasser.

### Zweites Beispiel

### Zweistufige Herstellung von DMPU

### a) Destillationsschritt 1

Der wie unter Beispiel 1 a) erhaltene DMPU-Rohaustrag wurde wiederum, wie bei Beispiel 1 unter b) beschrieben, einer Destillationskolonne zugeführt und dort in einer zweistufigen Ausführungsform destilliert. Dabei wurden im ersten Destillationsschritt die Leichtsieder, wie Ameisensäure und Wasser, abgetrennt und im zweiten Destillationsschritt das gewünschte Endprodukt von Neben- und Hochsiedern befreit. Die Reaktionsparameter wurden, wie nachfolgend angegeben, eingestellt:

| Destillationsschritt 1: | |
|---|---|
| Temperatur Zulauf | 150°C |
| Temperatur Kopf | 107-110°C |
| Temperatur Sumpf | 225°C |
| Kopfvakuum | 200 mbar |
| Differenzdruck | 20-30 mbar |
| Rücklauf/Zulauf | 1:1,75 |
| Zusammensetzung Sumpfablauf | 94 % DMPU, 5 % Nebensieder, < 0,1 % Ameisensäure, < 0,1 % Wasser. |

### b) Destillationsschritt 2:

Folgende Destillationsparameter wurden für den zweiten Destillationsschritt eingestellt:

| Destillationsschritt 2: | |
|---|---|
| Temperatur Zulauf | 135°C |
| Temperatur Kopf | 121-125°C |
| Temperatur Sumpf | 170°C |
| Kopfvakuum | 200 mbar |
| Differenzdruck | 20-30 mbar |
| Rücklauf/Zulauf | 1:1,5 |
| Zusammensetzung des Endproduktes am Seitenabzug | > 99,5 % DMPU, < 0,4 % Nebensieder |

## Patentansprüche

1. Verfahren zur Herstellung von N-Alkyl-N'-methylalkylenharnstoffen der Formel 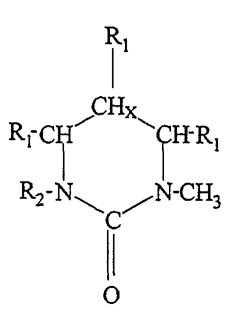
worin R₁ = H oder CH₃, R₂ = Cₙ H₂ₙ₊₁ mit n = 1-4 und x = 0 oder 1, mit einem Ameisensäuregehalt im Endproduckt von wenigen als 0,1% aus den entsprechenden Alkylenharnstoffen durch Umsetzung mit monomerem oder polymerisiertem Formaldehyd in Gegenwart von Ameisensäure, **dadurch gekennzeichnet, daß** das bei der Umsetzung erhaltene Gemisch aus N-Alkyl-N'-methylalkylenharnstoff und Amei-sensäure dem oberen Bereich einer Destillationskolonne zugeführt, ohne weitere Zusätze destilliert und dem unteren Bereich der Kolonne im wesentlichen Ameisensäure-freier N-Alkyl-N'-methylalkylenharnstoff entnommen wird, wobei der Druck im oberen Bereich der Kolonne höher als im unteren Bereich der Kolonne eingestellt wird und der Differenz-druck zwischen dem oberen und dem unteren Bereich der Kolonne 10 bis 100 mbar beträgt und die Temperatur im unteren Bereich der Kolonne höher als im oberen Bereich der Kolonne eingestellt wird, mit einer Temperaturdifferenz zwischen dem oberen und dem unteren Bereich der Kolonne von 40°C bis 210°C.

2. Verfahren nach Anspruch 1, **dadurch gekennzeichnet, daß** der Differenzdruck zwischen oberem und unterem Bereich der Kolonne 20 bis 40 mbar und die Temperaturdifferenz 80°C bis 150°C beträgt.

3. Verfahren nach Anspruch 1 oder 2, **dadurch gekennzeichnet, daß** der N-Alkyl-N'-methylalkylenharnstoff dem unteren Bereich der Kolonne gasförmig entnommen wird.

4. Verfahren nach einem der Ansprüche 1 bis 3, **dadurch gekennzeichnet, daß** die Kolonne eine Packung mit zumindest etwa 30 theoretischen Böden aufweist.

5. Verfahren nach einem der Ansprüche 1 bis 4, **dadurch gekennzeichnet, daß** der entsprechende Alkylenharnstoff mit dem Formaldehyd und der Ameisensäure bei einer Temperatur von 70 bis 150°C reagieren gelassen wird und anschließend zunächst ein dabei entstehendes Gemisch aus Ameisensäure und Wasser abdestilliert wird.

6. Verfahren nach einem der Ansprüche 1 bis 5 zur Herstellung von N, N'-Dimethylpropylenharnstoff.

7. Verfahren nach einem der Ansprüche 1 bis 5 zur Herstellung von N, N'-Dimethylethylenharnstoff.

## Claims

1. A process for preparing N-alkyl-N'-methylalkyleneureas of the formula 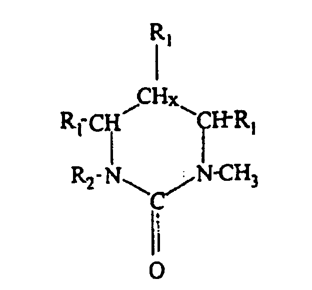
where R₁ = H or CH₃, R₂ = CₙH₂ₙ₊₁ with n = 1-4 and x = 0 or 1, with a formic acid content in the final product of less than 0.1% from the corresponding alkyleneureas by reaction with monomeric or polymerized formaldehyde in the presence of formic acid, which comprises feeding the mixture, obtained in the reaction, of N-alkyl-N'-methylalkyleneurea and formic acid to the upper region of a distillation column, distilling without further additions and removing essentially formic acid-free N-alkyl-N'-methylalkyleneurea in the lower region of the column, where the pressure in the upper region of the column is set at a higher level than in the lower region of the column, and the difference in pressure between the upper and lower regions of the column is from 10 to 100 mbar, and the temperature in the lower region of the column is set at a higher level than in the upper region of the column, with the difference in temperature between the upper and lower regions of the column being from 40°C to 210°C.

2. A process as claimed in claim 1, wherein the difference in pressure between the upper and lower regions of the column is from 20 to 40 mbar and the difference in temperature is from 80°C to 150°C.

3. A process as claimed in claim 1 or 2, wherein the N-alkyl-N'-methylalkyleneurea is removed in the form of a gas from the lower region of the column.

4. A process as claimed in any of claims 1 to 3, wherein the column has a packing with at least about 30 theoretical plates.

5. A process as claimed in any of claims 1 to 4, wherein the appropriate alkyleneurea is reacted with the formaldehyde and the formic acid at a temperature of from 70 to 150°C, and subsequently a mixture, produced therein, of formic acid and water is firstly distilled off.

6. A process as claimed in any of claims 1 to 5 for preparing N,N'-dimethylpropyleneurea.

7. A process as claimed in any of claims 1 to 5 for preparing N,N'-dimethylethyleneurea.

## Revendications

1. Procédé de préparation de N-alkyl-N'-méthylalkylène-urées de la formule : 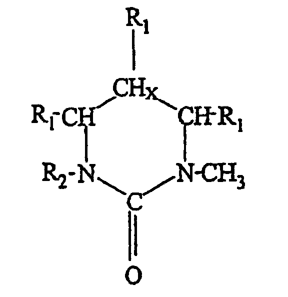
dans laquelle R₁ = H ou CH₃, R₂ = Cₙ H₂ₙ₊₁ où n = 1-4 et x = 0 ou 1, présentant une teneur en acide formique dans le produit final inférieure à 0,1%,
à partir des alkylèneurées correspondantes, par réaction avec du formaldéhyde monomère ou polymérisé en présence d'acide formique, **caractérisé en ce que** le mélange obtenu lors de la réaction et à base de N-alkyl-N'-méthylalkylèneurée et d'acide formique est amené à la zone supérieure d'une colonne de distillation et est distillé sans autre additif et **en ce qu'**à la zone inférieure de la colonne, de la N-alkyl-N'-méthylalkylèneurée sensiblement exempte d'acide formique est prélevée, la pression dans la zone supérieure de la colonne étant ajustée supérieure à celle de la zone inférieure de la colonne, la pression différentielle entre la zone supérieure et la zone inférieure de la colonne étant de 10 à 100 mbars et la température dans la zone inférieure de la colonne étant ajustée supérieure à celle de la zone supérieure de la colonne, avec une différence de température entre la zone supérieure et la zone inférieure de la colonne de 40°C à 210°C.

2. Procédé suivant la revendication 1, **caractérisé en ce que** la pression différentielle entre la zone supérieure et la zone inférieure de la colonne est de 20 à 40 mbars et la différence de température de 80°C à 150°C.

3. Procédé suivant l'une des revendications 1 et 2, **caractérisé en ce que** la N-alkyl-N'-méthylalkylèneurée est prélevée à la zone inférieure de la colonne sous forme gazeuse.

4. Procédé suivant l'une des revendications 1 à 3, **caractérisé en ce que** la colonne présente un gamissage comportant au moins environ 30 plateaux théoriques.

5. Procédé suivant l'une des revendications 1 à 4, **caractérisé en ce que** l'alkylèneurée correspondante est laissée à réagir avec le formaldéhyde et l'acide formique à une température de 70 à 150°C et **en ce qu'**ensuite un mélange formé d'acide formique et d'eau est tout d'abord séparé par distillation.

6. Procédé suivant l'une des revendications 1 à 5, pour la préparation de N,N'-diméthylpropylèneurée.

7. Procédé suivant l'une des revendications 1 à 5, pour la préparation de N,N'-diméthyléthylèneurée.
